(19) Europäisches Patentamt
European Patent Office
Office européen des brevets

(11) Publication number: 0 086 590
A1

# (12) EUROPEAN PATENT APPLICATION

(21) Application number: **83300495.5**

(22) Date of filing: **01.02.83**

(51) Int. Cl.³: **C 07 D 487/04**, A 61 K 31/40
//
C07C103/48 ,(C07D487/04,
209/00, 205/00),(A61K31/40,
31/43),(A61K31/40, 31/545)

(30) Priority: **17.02.82 GB 8204609**

(43) Date of publication of application: **24.08.83**
**Bulletin 83/34**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Coulton, Steven, 6 Badgers Close, Horsham West Sussex (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Antibacterial agents, their preparation and use.**

(57) The invention provides a compound of formula (II):

$R^1$–CO–O–CHR$^2$–O–CO–R$^3$ (II)

or a salt or ester therof:
wherein $R^1$ is a substituted or unsubstituted 7-oxo-1-azabicyclo[3.2.0]hept-2-ene ring system, $R^2$ is hydrogen or a hydrocarbon moiety, and $R^3$ is the residue of a renal dipeptidase inhibitor $R^3CO_2H$. A process for the preparation of compounds of formula II and pharmaceutical compositions containing these compounds are also described.

EP 0 086 590 A1

# ANTIBACTERIAL AGENTS, THEIR PREPARATION AND USE

This invention relates to novel 7-oxo-1-azabicyclo [3.2.0]hept-2-ene derivatives and in particular to such derivatives with a C-2 esterified carboxy substituent. This invention further relates to processes for their preparation and to compositions containing them. These derivatives are of use in the treatment of bacterial infection.

European Patent Application Publication Numbers: 0001627, 0001628, 0017992 and 0030032 disclose synthetic antibacterial agents of the formula (I):

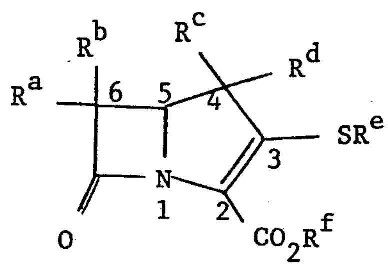

(I)

wherein $R^a$, $R^b$, $R^c$, $R^d$, $R^e$ and $R^f$ may be selected from a wide range of substituents.

It is reported (European Application Publication 0007614) that compounds of this type when administered in pharmaceutical compositions benefit from the inclusion of a renal dipeptidase inhibitor.

The present invention provides a compound of the formula (II):

$$R^1-CO-O-CHR^2-O-CO-R^3 \quad (II)$$

wherein $R^1$ is a substituted or unsubstituted 7-oxo-1-azabicyclo[3.2.0]hept-2-ene ring system, $R^2$ is hydrogen or a hydrocarbon moiety, and $R^3$ is the residue of a renal dipeptidase inhibitor $R^3CO_2H$.

Suitably $R^1$ is a group of the formula (III):

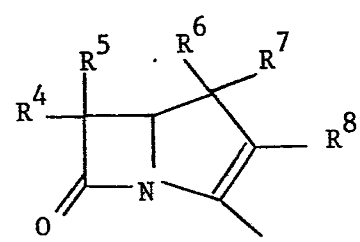

(III)

wherein $R^4$, $R^5$, $R^6$ and $R^7$ each represent a hydrogen atom or an organic group bonded <u>via</u> a carbon atom to the carbapenem ring, or $R^6$ and $R^7$ are joined so as to form together with the carbon atom to which they are attached an optionally substituted $C_{3-7}$ cycloalkyl ring, and $R^8$ is a hydrogen atom a group -SH, or a group $-S(O)_nR^9$ wherein n is zero or one and $R^9$ is an organic group bonded <u>via</u> a carbon atom to the sulphur atom.

Suitably $R^8$ is a hydrogen atom. Suitably $R^8$ is a group -SH. More suitably $R^8$ is a group $-S(O)_nR^9$ wherein aptly n is one but preferably n is zero.

Suitably the groups $R^4$, $R^5$, $R^6$, $R^7$ and $R^9$ in the formula (III) may be independently selected from $C_{1-6}$ alkyl, $C_{3-7}$ cycloalkyl, $C_{3-7}$ cycloalkyl ($C_{1-6}$) alkyl, $C_{2-6}$ alkenyl, $C_{2-6}$ alkynyl, aryl ($C_{1-6}$) alkyl, $C_{1-6}$ alkanoyl, aryl ($C_{1-6}$) alkanoyl, arylcarbonyl, heteroarylcarbonyl, aryl, heterocyclyl, heterocyclyl ($C_{1-6}$) alkyl, heteroaryl ($C_{1-6}$) alkyl or heteroaryl group, any of such groups being optionally substituted. Suitably the hetero atom or hetero atoms in the above named heteroaryl and/or heterocyclyl moieties are selected from 1 to 4 oxygen, nitrogen or sulphur atoms.

Suitable optional substituents for the groups $R^4$, $R^5$, $R^6$, $R^7$ and $R^9$ include $C_{1-6}$ alkyl, amino, $C_{1-6}$ alkanoylamino, mono, di- and tri- ($C_{1-6}$) alkylamino, hydroxy, $C_{1-6}$ alkoxy, mercapto, $C_{1-6}$ alkylthio, heteroarylthio, arylthio, sulphamoyl, carbamoyl, amidino, guanidino, nitro, chloro, bromo, fluoro, carboxy and salts and esters thereof, $C_{1-6}$ alkanoyloxy, arylcarbonyl and heteroaryl- carbonyl.

Suitably $R^9$ is $C_{1-6}$ alkyl, $C_{2-6}$ alkenyl, aryl such as phenyl, aralkyl wherein the aryl moiety is preferably phenyl and the alkyl part has 1 to 6 carbon atoms, for example benzyl, phenethyl; heterocyclyl or heterocyclylalkyl wherein the alkyl part has 1 to 3 carbon atoms and the heterocyclic ring comprises 4 to 7 atoms, preferably 5 to 6, up to 4 of which may be selected from oxygen, sulphur and nitrogen, such as pyridyl, furyl, thienyl, pyrimidinyl, imidazolyl, triazinyl and tetrazolyl.

Preferably $R^9$ is $C_{1-6}$ alkyl for example methyl, ethyl or propyl, optionally substituted by amino, $C_{1-6}$ alkanoylamino, carboxy, mono- and di- $C_{1-6}$-alkylamino,

hydroxy, amidino or $C_{1-6}$ alkoxy; $C_{2-6}$ alkenyl such as vinyl optionally substituted with $C_{1-6}$ alkanoylamino such as acetamido; or is an optionally substituted phenyl, pyrimidinyl or pyridyl group.

Preferably in the groups of the formula (III) either $R^4$ or $R^5$ is a hydrogen atom. In an alternative aspect both $R^4$ and $R^5$ are hydrogen atoms.

More suitably one of $R^4$ and $R^5$ is a hydrogen atom and the other is selected from α-sulphonato-oxyethyl, α-sulphonato-oxyprop-2-yl or a group $CR^{9'}R^{10}R^{11}$ wherein $R^{9'}$ is a hydrogen atom or a hydroxy group, $R^{10}$ is a hydrogen atom or a $C_{1-6}$ alkyl group, $R^{11}$ is a hydrogen atom, a $C_{1-6}$ alkyl group, a benzyl group, a phenyl group, or is joined to $R^{9'}$ to form together with the carbon atom to which they are joined a carbocylic ring of 5 to 7 carbon atoms.

Most suitably $R^{9'}$ is a hydrogen atom or a hydroxy group. Most suitably $R^{11}$ is a hydrogen atom or a methyl, ethyl, n-propyl or phenyl group. Most suitably $R^{10}$ is a hydrogen atom or a methyl, ethyl or n-propyl group. Favourably $R^{10}$ is a hydrogen atom or methyl group. Favourably $R^{11}$ is a hydrogen atom or methyl group.

Preferably the $CR^{9'}R^{10}R^{11}$ moiety is a $-C(CH_3)_2OH$, $-CH(CH_3)OH$, $-CH(C_2H_5)OH$, $-CH_2CH_3$ or $-CH(CH_3)_2$ group, of these the $-CH(CH_3)OH$ group is most favoured.

It is to be realised that the groups of the formula (III) wherein $R^{9'}$, $R^{10}$ and $R^{11}$ have different values may exist in either the 8R or 8S form (the C-8 carbon atom being that adjacent to the carbon atom of

the carbapenem ring). If desired these compounds may be presented as the 8R or the 8S isomer or as a mixture thereof.

The groups of the formula (III) wherein one of $R^4$ and $R^5$ (but not both) is hydrogen may have the cis- or trans- configuration about the β-lactam ring, or may be provided as mixtures thereof.

Suitably $R^6$ and $R^7$ may be independently selected from $C_{1-6}$ alkyl such as methyl, ethyl or n-propyl; aryl ($C_{1-6}$) alkyl such as benzyl or phenethyl; or $C_{3-7}$ cycloalkyl such as cyclohexyl. In another aspect $R^6$ and $R^7$ may be joined so as to form with the carbon atom to which they are attached a $C_{3-7}$ cycloalkyl ring.

Preferably $R^6$ is $C_{1-6}$ alkyl in particular methyl and $R^7$ is $C_{1-6}$ alkyl in particular methyl.

Suitably in the compounds of the formula (II) $R^2$ is a hydrogen atom or a $C_{1-6}$ alkyl, aryl or aryl ($C_{1-6}$) alkyl group. More suitably $R^2$ is hydrogen, methyl, ethyl, benzyl or phenyl. Preferably $R^2$ is a hydrogen atom.

$R^3$ is the residue of a renal dipeptidase inhibitor $R^3CO_2H$ such as those known in the art. For example $R^3$ may have the formula (IV):

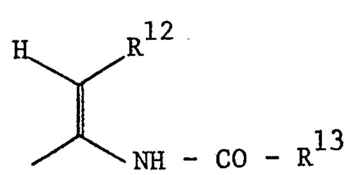

(IV)

wherein $R^{12}$ is a substituted or unsubstituted $C_{1-15}$ hydrocarbon and $R^{13}$ is a substituted or unsubstituted $C_{3-10}$ hydrocarbon.

Suitable substituents for the groups $R^{12}$ and $R^{13}$ include amino, $C_{1-6}$ alkanoylamino, mono- and di-$(C_{1-6})$ alkylamino, $C_{1-6}$ alkoxy, $C_{1-6}$ alkylthio, hydroxy, mercapto, arylthio such as carboxypyridyl, sulphamoyl, carbamoyl, amidino, guanidino, chloro, bromo, fluoro, carboxy and salts and esters thereof, $C_{1-6}$ alkanoyloxy, arylcarbonyl, heteroarylcarbonyl and β-amino-β-carboxy-ethylthio.

Suitably $R^{12}$ is $C_{1-6}$ alkyl such as methyl, ethyl, n-propyl, isopropyl, isobutyl or n-pentyl, or $R^{12}$ is $C_{3-7}$ cycloalkyl such as cyclopropyl. In a further aspect $R^{12}$ may be $C_{3-7}$ cycloalkyl$(C_{1-6})$alkyl such as cyclopropylmethyl.

Preferably $R^{12}$ is methyl.

Suitably $R^{13}$ is $C_{3-8}$ alkyl such as n-propyl, isopropyl, n-butyl, isobutyl, n-pentyl or isohexyl; or $R^{13}$ is $C_{3-7}$ cycloalkyl such as cyclopropyl. In a further aspect $R^{13}$ may be a $C_{3-7}$ cycloalkyl$(C_{1-6})$alkyl moiety, for example cyclopropylmethyl. In another favoured aspect $R^{13}$ may be cyclopropyl optionally substituted by one or two alkyl groups, for example dimethylcyclopropyl.

A preferred sub-group of compounds is that of the formula (V):

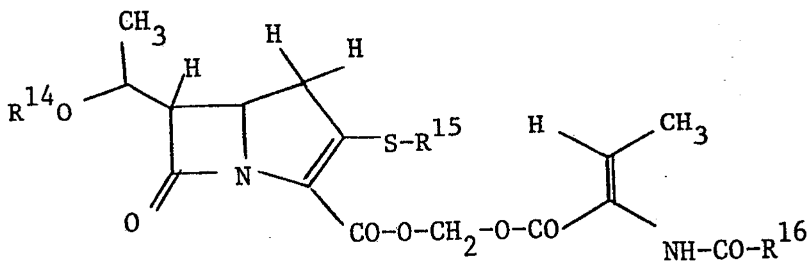

(V)

wherein $R^{14}$ is hydrogen or $HO_3S-$ or a pharmaceutically acceptable salt thereof, $R^{15}$ is $C_{1-6}$ alkyl optionally substituted by amino, $C_{1-6}$ alkanoylamino, carboxy, mono- and di- $C_{1-6}$-alkylamino, hydroxy, amidino or $C_{1-6}$ alkoxy; $C_{1-6}$ alkanoylaminoethenyl such as acetamidoethenyl, or optionally substituted phenyl, pyridyl or pyrimidinyl, and $R^{16}$ is $C_{3-6}$ alkyl such as isobutyl.

If a carboxy group is present as a substituent in any of the compounds of this invention, then this may be presented as the free acid or in salified or esterified form. Such salts would suitably be in pharmaceutically acceptable form and include alkali metal salts such as sodium or potassium, and alkaline earth metal salts such as calcium or magnesium. In addition pharmaceutically acceptable salts may be formed at the C-6-α-sulphonato-oxyethyl or α-sulphonato-oxyprop-2-yl substituent, if present. Such salts would preferably be alkali metal salts such as sodium or potassium, or substituted ammonium salts. Non-pharmaceutically acceptable salts would also be of use as they may be converted to the pharmaceutically acceptable salts of the compounds of the invention.

Suitable esterifying groups for any carboxy group present in the compounds of this invention include those convertible to the free acid or salt thereof by biological methods such as *in-vivo* hydrolysis or

enzymic hydrolysis, and those cleavable by chemical methods such as hydrogenolysis, hydrolysis, electrolysis or photolysis.

Suitable esterifying groups include those of the sub-formulae (a) - (f):

$-CHA^1A^2$ (a)

$-CHA^3A^4$ (b)

$-CHA^5-OCOA^6$ (c)

$-CHA^5-OA^7$ (d)

$-SiA^8A^9A^{10}$ (e)

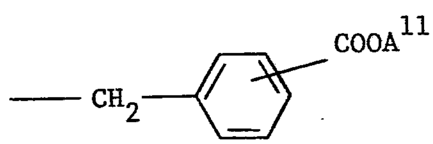
(f)

wherein $A^1$ is a hydrogen atom, $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacylmethyl or bromophenacylmethyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable <u>in-vivo</u> hydrolysable esters are those esters which hydrolyse in the human body to produce the parent acid or its salt. Such esters may be identified by administration to a test

animal such as a rat or mouse by intravenous administration and thereafter examining the test animal's body fluids for the presence of the compound of the formula $R^1CO_2H$ or its salt.

Suitable esters of this type include those of sub-formula (c) as hereinbefore defined.

The compounds of this invention may be employed in the treatment of bacterial infection. Thus the present invention also provides a pharmaceutical composition which comprises a compound of the formula (II) or a pharmaceutically acceptable salt or ester thereof and a pharmaceutically acceptable carrier. Preferably any such ester is <u>in-vivo</u> hydrolysable.

The compositions of this invention may be prepared by conventional methods of preparing antibiotic compositions and in conventional manner may be adapted for oral, or parenteral administration.

Aptly, the compositions of this invention are in the form of a unit-dose composition adapted for oral administration.

Alternatively the compositions of this invention are in the form of a unit-dose composition adapted for administration by injection.

Unit dose forms according to this invention will normally contain from 50 to 1000 mg of a compound of this invention, for example about 62.5, 100, 150, 200, 250, 500 or 750 mg. Such compositions may be administered from 1 to 6 times a day or more

conveniently 2, 3 or 4 times a day so that the total daily dose for a 70 kg adult is about 200 to 2000 mg, for example about 400, 600, 750, 1000 or 1500 mg.

The compositions of this invention may be used to treat bacterial infection in animals such as mammals including humans, for example infections of the respiratory tract, urinary tract or soft tissues, or mastitis in cattle.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth, potato starch or polyvinylpolypyrrolidone, magnesium stearate or sodium lauryl sulphate.

Orally administrable forms of the compositions of this invention are most suitably in the form of unit-dose units such as tablets or capsules.

One feature of the compounds of the present invention is that they are believed to break down <u>in-vivo</u> to liberate the moieties of the antibacterial 7-oxo-1-azabicyclo[3.2.0]hept-2-ene moiety and the renal dipeptidase inhibitor.

The present invention also provides synergistic pharmaceutical compositions which comprise a pharmaceutical composition as hereinbefore described and a penicillin or cephalosporin.

Suitable penicillins for inclusion in the synergistic compositions of this invention include benzylpenicillin, phenoxymethylpenicillin, ampicillin, amoxycillin, ticarcillin, suncillin, sulbenicillin, azlocillin or mezlocillin; in particular amoxycillin as its sodium salt or trihydrate is preferred.

Suitable cephalosporins for inclusion in the synergistic compositions of this invention include cephaloridine, cefazolin and cephradine.

The penicillin or cephalosporin is generally utilised in its conventionally administered amount.

The weight ratio between the compound of this invention and penicillin or cephalosporin is generally from 10:1 to 1:10, more usually from 5:1 to 1:5 and normally from 3:1 to 1:3.

In another aspect the present invention provides a process for the preparation of a compound of the formula (II) which comprises the reaction of a compound of the formula (VI):

$$R^1CO_2H \qquad (VI)$$

or salt or other reactive derivative thereof, wherein $R^1$ is as hereinbefore defined, with a compound of the formula (VII):

$$Y\text{-}CHR^2\text{-}O\text{-}CO\text{-}R^3 \qquad (VII)$$

wherein $R^2$ and $R^3$ are as hereinbefore defined and Y is a displaceable group.

Suitably Y is a readily displaceable group such as a halo atom, for example chloro, iodo or bromo, preferably iodo.

The reaction is suitably performed in a substantially inert organic solvent known to be convenient for esterification reactions, such as dimethylformamide, acetone, ethyl acetate or a halogenated hydrocarbon. Preferably such reaction is performed at a non-extreme temperature such as 0° to 50°C, preferably at ambient temperature.

Suitably the compound of the formula (VI) is reacted in the form of its salt, which need not be pharmaceutically acceptable. Suitable salts include alkali metal salts such as lithium, sodium or potassium.

The compounds of the formula (VII) may be prepared by the reaction of a compound of the formula (VIII):

$$Y\text{-}CHR^2\text{-}Z \qquad (VIII)$$

wherein Y and $R^2$ are as hereinbefore defined, and Z is a better leaving group than Y; with a compound of the formula (IX):

$$R^3\text{-}CO\text{-}OH \qquad (IX)$$

or a salt thereof. Such a reaction may be performed under similar conditions to those of the reaction between compounds of the formulae (VI) and (VII). Suitably Z is a halo atom for example, chloro, iodo or bromo, or is a sulphonate moiety such as $C_{1-6}$ alkylsulphonyloxy or arylsulphonyloxy for example, p-toluenesulphonate.

If desired a group Y in the compound of the formula (VII) may be converted to another group Y in conventional manner, for example, chloro may be converted to iodo with sodium iodide in acetone.

In a further aspect the present invention provides a process for the preparation of a compound of the formula (II) which comprises the reaction of a compound of the formula (X):

$$R^1CO\text{-}O\text{-}CHR^2\text{-}Y \qquad (X)$$

wherein $R^1$, $R^2$ and Y are as hereinbefore defined, with a compound of the formula (XI):

$$HO\text{-}CO\text{-}R^3 \qquad (XI)$$

or a salt or other reactive derivative thereof, wherein $R^3$ is as hereinbefore defined.

Suitably in the above reaction Y is chloro, iodo or bromo, preferably iodo. The reaction may be performed in similar manner to that described between compounds of the formulae (VI) and (VII).

The compounds of the formula (X) may be prepared by the reaction of a compound of the formula (VI) with a compound of the formula (VIII), under analogous conditions to those described for the reaction of compounds (VIII) and (IX).

In one suitable aspect a compound of the formula (VI) is reacted with chloromethylchlorosulphate in aqueous dichloromethane in the presence of bicarbonate

and a phase transfer catalyst (eg $Bu_4NHSO_3$), and the resultant chloromethyl ester is converted to an iodomethyl ester with sodium iodide in acetone.

The following Examples illustrate the invention.

DESCRIPTION 1

Chloromethyl ester of Z-2-isovaleramido-2-butenoic acid

Z-2-Isovaleramido-2-butenoic acid was converted to its sodium salt by the addition of one molar equivalent of sodium hydrogen carbonate to a suspension of the acid in water. Lyophilisation afforded the sodium salt as a white solid.

The sodium salt of Z-2-isovaleramido-2-butenoic acid (4.5 gm) was dissolved in dry dimethylformamide (50 ml) and stirred at room temperature for 16 hours with chloroiodomethane (9.59 gm; 2.5 equiv.). The solvent was evaporated at reduced pressure and the residue partitioned between ethyl acetate and water. The organic layer was washed with saturated sodium chloride solution, dried over anhydrous magnesium sulphate, filtered and evaporated at reduced pressure to yield the crude product as a pale yellow oil. This oil was chromatographed over silica gel (200 gm) eluting with a gradient of 25 to 50% ethyl acetate/hexane. The pure chloromethyl ester of Z-2-isovaleramido-2-butenoic acid was obtained as a white solid after trituration with hexane (0.225 gm), $\nu_{max}$ ($CHCl_3$) 3410, 1728, 1682, 1655 $cm^{-1}$; $d_H$ ($CDCl_3$) 1.02 (6H, d, $(C\underline{H}_3)_2CH$), 1.83 (3H, d, vinylic $C\underline{H}_3$), 2.05-2.30 (3H, m, $CHC\underline{H}_2CO+C\underline{H}CH_2CO$), 5.79 (2H, s, $CO_2C\underline{H}_2Cl$), 6.84 (1H, broad s, $N\underline{H}$), 6.94 (1H, q, vinylic $\underline{H}$).

## DESCRIPTION 2

## Iodomethyl ester of Z-2-isovaleramido-2-butenoic acid

The chloromethyl ester of Z-2-isovaleramido-2-butenoic acid (190 mg) and sodium iodide (247 mg) were dissolved in dry acetone (25 ml). The solution was stirred at room temperature for 16 hours and then refluxed for 3 hours. After cooling the solvent was evaporated at reduced pressure and the residue partitioned between ethyl acetate and water. The organic layer was washed with dilute sodium thiosulphate solution, saturated sodium chloride solution, dried over anhydrous magnesium sulphate and evaporated at reduced pressure to yield a white solid, which was chromatographed over silica gel (5 gm). Elution with ethyl acetate afforded the iodomethyl ester of Z-2-isovaleramido-2-butenoic acid as a white solid (150 mg), $d_H$ ($CDCl_3$) 1.0 (6H, $(C\underline{H}_3)_2CH$), 1.80 (3H, d, vinylic $C\underline{H}_3$), 1.90-2.35 (3H, m), 6.0 (2H, s, $CO_2C\underline{H}_2I$), 6.88 (1H, q, vinylic $\underline{H}$), 7.35 (1H, broad res., NH).

EXAMPLE 1

2-Isovaleramidobut-2Z-enoyloxymethyl (5R,6R)-3-[(E)-2-acetamidoethenylthio]-6-[(S)-1-hydroxyethyl]-7-oxo-1-azabicyclo[3.2.0]hept-2-ene-2-carboxylate

Sodium (5R,6R)-3-[(E)-2-acetamidoethenylthio]-6-[(S)-1-hydroxyethyl]-7-oxo-1-azabicyclo[3.2.0]hept-2-ene-2-carboxylate (155 mg; 0.46 mM) was dissolved in dry dimethylformamide (5 ml) and stirred at room temperature for two hours with the iodomethyl ester of Z-2-isovaleramido-2-butenoic acid (75mg; 0.23 mM). The reaction mixture was then partitioned between ethyl acetate and water. The organic layer was washed with dilute sodium thiosulphate solution, saturated sodium chloride solution, dried over anhydrous magnesium sulphate and the solvent removed at reduced pressure to yield the crude product, which was chromatographed over silica gel (5 gm). Elution with a gradient of 0 to 10% ethanol/chloroform afforded the pure 2-isovaleramidobut-2Z-enoyloxymethyl (5R,6R)-3-[(E)-2-acetamidoethenylthio]-6-[(S)-1-hydroxyethyl]-7-oxo-1-azabicyclo[3.2.0]hept-2-ene-2-carboxylate (24 mg) as a pale yellow solid, $\lambda_{max}$ (EtOH) 325 nm ($\varepsilon$m 12,880), 227 nm ($\varepsilon$m 17,490), $\nu_{max}$ ($CHCl_3$) 3420, 3300-3400 (broad), 1778, 1730 (shoulder), 1700, 1622 $cm^{-1}$; $d_H$ ($CDCl_3$) 1.01 (6H, d, $(C\underline{H}_3)_2CH$), 1.34 (3H, d, $C\underline{H}_3CH(OH)$), 1.79 (3H, d, vinylic $CH_3$), 2.08 (3H, s, $COC\underline{H}_3$), 2.10-2.25 (3H, m, $CHC\underline{H}_2CO+C\underline{H}CH_2CO$), 3.0-3.15 (1H, dd, 4-$C\underline{H}_a$), 3.28-3.42 (1H, dd, 4-$C\underline{H}_b$), 3.54 (1H, dd, $\underline{J}$ 5.5Hz and 9.0Hz, 6-C$\underline{H}$), 4.03-4.16 (1H, m, 8-C$\underline{H}$), 4.16-4.30 (1H, dt, 5-C$\underline{H}$), 5.82 (1H, d, $\underline{J}$ 13.5Hz, CH=C$\underline{H}$-S), 5.92 (2H, AB, $CO_2C\underline{H}_2O$), 6.93 (1H, q, $\underline{H}C=C-CO_2$), 7.21 (1H, dd, $\underline{J}$ 10.0 and 13.5Hz, C=C$\underline{H}$NH), 7.36 (1H, s, N$\underline{H}$), 8.91 (1H, d, N$\underline{H}$).

Table 1 shows representative biological data for the compound produced in Example 1.

Table 1

| Organism | mic (μg/ml) |
|---|---|
| Enterobacter cloacae N1 | 6.2 |
| Escherichia coli 0111 | 0.8 |
| JT39 R+ | 25 |
| ESS | 0.2 |
| E8 | 0.8 |
| E96 R+ | 100 |
| Klebsiella aerogenes A | 6.2 |
| Proteus mirabilis 977 | 3.1 |
| 13 | 0.8 |
| Proteus morganii 1580 | 25 |
| Proteus rettgeri WM16 | 6.2 |
| Pseudomonas aeruginosa A | >100 |
| 11 | 100 |
| Dalgleish | 100 |
| Serratia marcescens US20 | 6.2 |
| Bacillus subtilis A | 0.4 |
| Staphylococcus aureus Oxford | 0.4 |
| Russell | 0.8 |
| Streptococcus faecalis I | 3.1 |
| Effect of serum on activity | 2-8 fold |

EXAMPLE 2

Sodium salt of 2-Isovaleramidobut-2Z-en-oyloxymethyl (5R,6R)-3-[(E)-2-acetamidoethenylthio]-6-[(S)-1-hydroxysulphonyloxyethyl]-7-oxo-1-azabicyclo[3.2.0]hept 2-ene-2-carboxylate

Disodium (5R,6R)-3-[(E)-2-acetamidoethenylthio]-6-[(S)-1-hydroxysulphonyloxyethyl]-7-oxo-1-azabicyclo [3.2.0]hept-2-ene-2-carboxylate (201 mg, 0.46 mM) was dissolved in dry dimethylformamide (5 ml) and stirred at room temperature for 1.5 hours with the iodomethyl ester ofZ-2-isovaleramido-2-butenoic acid (75 mg, 0.23 mM). The solvent was then reduced to small volume at reduced pressure and applied to a column of silica gel (6 gm) in chloroform. Elution with a gradient of 0 to 30% ethanol/chloroform afforded the crude product, which was rechromatographed over silica gel (3 gm). Elution with a gradient of 0 to 20% ethanol/chloroform gave the pure sodium salt of 2-isovaleramidobut-2Z-enoyloxymethyl (5R,6R)-3-[(E)-2-acetamidoethenylthio]-6-[(S)-1-hydroxysulphonyloxyethyl]-7-oxo-1-azabicyclo [3.2.0]hept-2-ene-2-carboxylate (45 mg) as a white solid, $\nu_{max}$ (KBr) 3440 (broad), 3260 (broad), 1760, 1685 (shoulder), 1662, 1620 $cm^{-1}$; $\lambda_{max}$ ($H_2O$) 330 nm ($\varepsilon$m 10,064), $d_H$ ($d_7$-DMF) 0.96 (6H, d, $(C\underline{H}_3)_2CHCH_2$), 1.44 (3H, d, $C\underline{H}_3CH(OH)$)), 1.77 (3H, d, vinylic $C\underline{H}_3$), 2.02 (3H, s, $COC\underline{H}_3$) 2.02-2.25 (3H, m, $C\underline{H}CH_2CO$ + $CHC\underline{H}_2CO$), 2.96-3.12 (1H, dd, 4-$C\underline{H}_a$), 3.71 (1H, dd, $\underline{J}$ 5.5 and 10.5Hz, 6-C$\underline{H}$), 3.78-3.93 (1H, dd, 4-$C\underline{H}_b$), 4.22-4.35 (1H, dt 8-C$\underline{H}$), 4.47-4.61 (1H, dt, 5-C$\underline{H}$), 5.94 (3H, d+AB, $CO_2C\underline{H}_2O$ + S-C$\underline{H}$=C), 6.60 (1H, q, vinylic $\underline{H}$), 7.21 (1H, dd, $\underline{J}$ 10.5 and 14.5Hz, C=C$\underline{H}$NH), 9.23 (1H, s, N$\underline{H}$), 10.69 (1H, d, N$\underline{H}$).

Table 2 shows representative biological data for the compound of Example 2.

Table 2

| Organism | mic (μg/ml) |
|---|---|
| Enterobacter cloacae N1 | 3.1 |
| Escherichia coli 0111 | 3.1 |
| JT39 R+ | 1.6 |
| ESS | 1.6 |
| E8 | 6.2 |
| E96 R+ | 12.5 |
| Klebsiella aerogenes A | 0.4 |
| Proteus mirabilis 977 | 3.1 |
| 13 | - |
| Proteus morganii 1580 | - |
| Proteus rettgeri WM16 | 0.8 |
| Pseudomonas aeruginosa A | 100 |
| 11 | 100 |
| Dalgleish | 100 |
| Serratia marcescens US20 | 6.2 |
| Bacillus subtilis A | 1.6 |
| Staphylococcus aureus Oxford | 1.6 |
| Russell | 3.1 |
| Streptococcus faecalis I | 50 |
| Effect of serum on activity | 2-4 fold |

EXAMPLE 3

2-Isovaleramidobut-2Z-enoyloxymethyl 5(R,S),6(S,R)-3-(pyrimidin-2-ylthio)-6-[1(R,S)-hydroxyethyl]-7-oxo-1-azabicyclo[3.2.0]hept-2-ene-2-carboxylate

The title compound was prepared as a white solid by the reaction of sodium 5(R,S),6(S,R)-3-(pyrimidin--2-ylthio)-6-[1(R,S)-hydroxyethyl]-7-oxo-1-azabicyclo[3.2.0]hept-2-carboxylate with the iodomethyl ester of Z-2-isovaleramido-2-butenoic acid, following the procedure described in example 1;
$\nu_{max}$ ($CHCl_3$) 3400, 1775, 1729, 1680, 1650 (shoulder), 1559, 1549 $cm^{-1}$; $\lambda_{max}$ (EtOH) 323 nm ($\varepsilon$m 12,192), 237 nm ($\varepsilon$m 9,931).

Table 3 shows representative biological data for the compound of Example 3.

Table 3

| Organism | mic (μg/ml) |
|---|---|
| Enterobacter cloacae N1 | 25 |
| Escherichia coli 0111 | 6.2 |
| JT39 R+ | 3.1 |
| ESS | 1.6 |
| Klebsiella aerogenes A | 3.1 |
| Proteus mirabilis 977 | 12.5 |
| Proteus morganii 1580 | 25 |
| Proteus rettgeri WM16 | 25 |
| Pseudomonas aeruginosa A | >100 |
| Dalgleish | >100 |
| Serratia marcescens NEU3974 | 100 |
| Bacillus subtilis A | 0.8 |
| Staphylococcus aureus Oxford | 0.8 |
| Russell | 0.8 |
| Streptococcus faecalis I | 50 |
| Effect of serum on activity | 10-20 fold |

It has been found that the above described compounds can be hydrolysed in vivo to liberate the antibacterial agent and the dipeptidase inhibitor. The liberation of the active components together can result in improved absorption, metabolic stability and urinary recovery of the antibacterial agent.

Table 4 shows mouse urinary recovery figures for the compounds of Examples 1 and 2 together with the figures when the equivalent parent antibacterial agents were administered alone. The results show that considerably more antibacterial agent is recovered when administered in the form of the compounds of Examples 1 and 2. By way of comparison. the parent antibacterial agent of the compound of Example 2 (ie sodium salt of [-2-acetamido-ethenylthio]-6-(1-hydroxysulphonyloxyethyl)-7-oxo-1-azabicyclo[3.2.0]hept-2-ene-2-carboxylic acid) was administered together with the corresponding dipeptidase inhibitor (ie 2-isovaleramidobut-2-ene) in a 1:1 relationship. The results show that approximately the same quantity of antibacterial agent was recovered. However, since the compound of Example 2 liberates an approximate ratio of 2:1 by weight of antibacterial agent to inhibitor, the compound of Example 2 makes much more efficient use of the dipeptidase inhibitor.

TABLE 4

Mouse Urinary Recoveries

| Compound | Example No | Dose | Urinary Recovery |
| --- | --- | --- | --- |
| 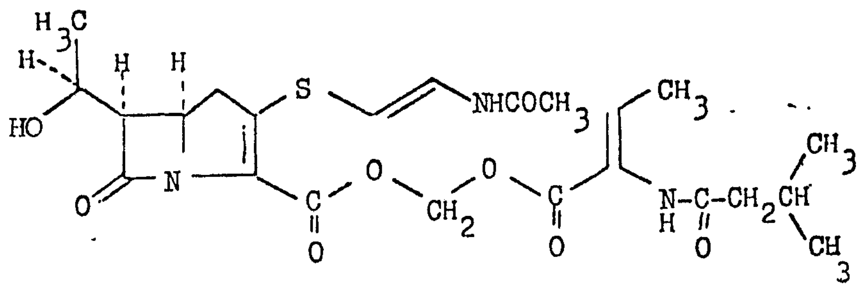 | 1 | 10 mg/kg s/c | 1.8% * |
| (A) 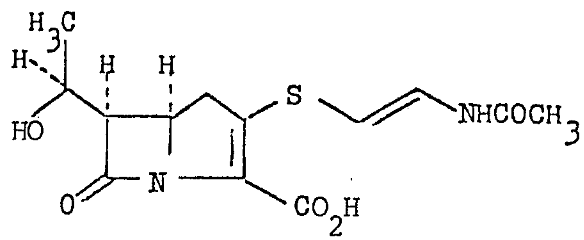 | | 10 mg/kg s/c | 0.3% |
| 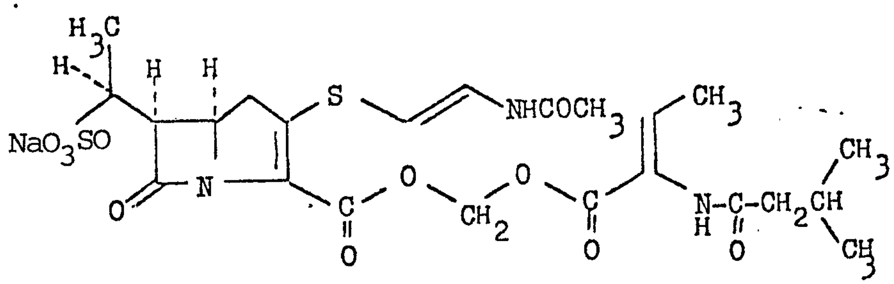 | 2 | 10 mg/kg s/c | 8.0% * |
| (B) 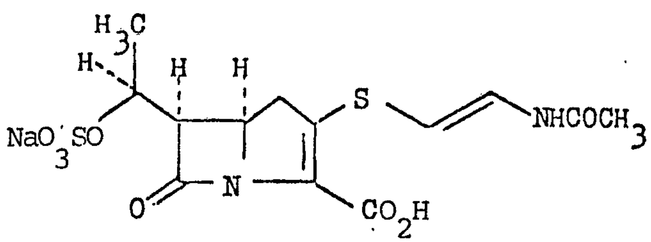 | | 10 mg/kg s/c | 1.5% |

TABLE 4 CONTINUED

| Compound | Example No | Dose | Urinary Recovery |
|---|---|---|---|
| 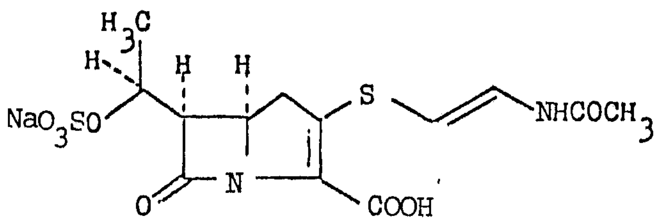 | - | 50 mg | |
| + | | + | 8.6% |
| 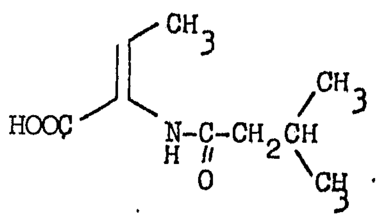 | - | 50 mg/kg s/c | |

* calculated urinary recovery based on proportion of antibacterial agent (ie $R^1H$) present in the total dose.

Further advantages of the present compounds are good shelf life and ease of administration.

CLAIMS:

1. A compound of formula (II):

$$R^1\text{-CO-O-CHR}^2\text{-O-CO-R}^3 \qquad \text{(II)}$$

or a salt or ester thereof:

wherein $R^1$ is a substituted or unsubstituted 7-oxo-1-azabicyclo[3.2.0]hept-2-ene ring system, $R^2$ is hydrogen or a hydrocarbon moiety, and $R^3$ is the residue of a renal dipeptidase inhibitor $R^3CO_2H$.

2. A compound according to claim 1 wherein $R^1$ is a group of formula (III):

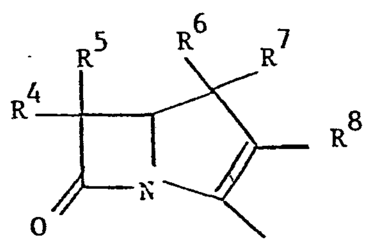

(III)

wherein $R^4$, $R^5$, $R^6$ and $R^7$ each represent a hydrogen atom or an organic group bonded via a carbon atom to the carbapenem ring, or $R^6$ and $R^7$ are joined so as to form together with the carbon atom to which they are attached an optionally substituted $C_{3-7}$ cycloalkyl ring, and $R^8$ is a hydrogen atom a group -SH, or a group $-S(O)_nR^9$ wherein n is zero or one and $R^9$ is an organic group bonded via a carbon atom to the sulphur atom.

3. A compound according to claim 1 or claim 2 wherein $R^3$ is a group of formula (IV):

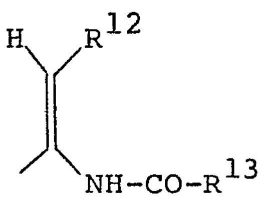

(IV)

wherein $R^{12}$ is a substituted or unsubstituted $C_{1-15}$ hydrocarbon and $R^{13}$ is a substituted or unsubstituted $C_{3-10}$ hydrocarbon.

4. A compound according to any one of the following preceding claims of formula (V)

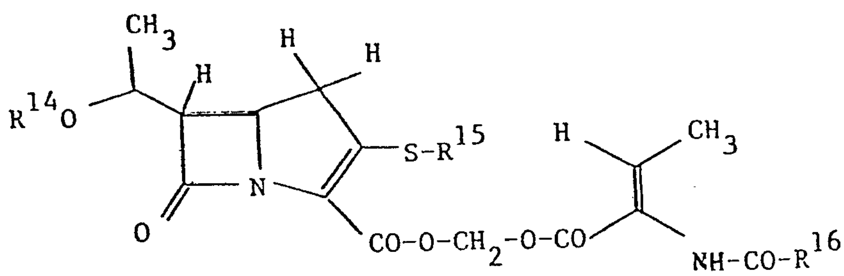

(V)

wherein $R^{14}$ is hydrogen or $HO_3S-$ or a pharmaceutically acceptable salt thereof, $R^{15}$ is $C_{1-6}$ alkyl optionally substituted by amino, $C_{1-6}$ alkanoylamino, carboxy, mono- and di- $C_{1-6}$-alkylamino, hydroxy, amidino or $C_{1-6}$ alkoxy; $C_{1-6}$ alkanoylaminoethenyl, or optionally substituted phenyl, pyridyl or pyrimidinyl, and $R^{16}$ is $C_{3-6}$ alkyl such as isobutyl.

5. A compound according to any one of the preceding claims selected from 2-isovaleramidobut-2Z-enoyloxymethyl (5R, 6R)-3-[(E)-2-acetamidoethenylthio]-6-[(S)-1-hydroxyethyl]-7-oxo-1-azabicyclo[3.2.0] hept-2-ene-2-carboxylate, sodium salt of 2-isovaleramidobut-2Z-enoyloxymethyl (5R, 6R)-3-[(E)-2-acetamidoethenylthio]-6-[(S)-1-hydroxysulphonyloxyethyl]-7-oxo-1-azabicyclo [3.2.0]hept-2-ene-2-carboxylate, and 2-isovaleramidobut-2Z-enoyloxymethyl 5(R,S), 6(S,R)-3-(pyrimidin-2-ylthio) -6-[1(R,S)-hydroxyethyl]-7-oxo-1-azabicyclo[3.2.0] hept -2-ene-2-carboxylate.

6. A process for producing a compound according to any one of the preceding claims which process comprises the reaction of a compound of formula VI:

$$R^1CO_2H \qquad VI$$

or a salt or derivative thereof, wherein $R^1$ is as defined in claim 1, with a compound of formula (VII):

$$Y\text{-}CHR^2\text{-}OCO\text{-}R^3 \qquad (VII)$$

wherein $R^2$ and $R^3$ are as defined in claim 1 and Y is a displaceable group such as halo.

7. A process for producing a compound according to any one of claims 1 to 5 which process comprises the reaction of a compound of formula (X):

$$R^1CO\text{-}O\text{-}CHR^2\text{-}Y \qquad (X)$$

wherein $R^1$ and $R^2$ are as defined in claim 1 and Y is a displaceable group such as halo with a compound of formula (XI)

$$HO\text{-}CO\text{-}R^3 \qquad (XI)$$

or a salt or derivative thereof, wherein $R^3$ is as defined in claim 1.

8. A pharmaceutical composition comprising a compound of formula (II) as defined in claim 1 or a pharmaceutically acceptable salt or ester thereof and a pharmaceutically acceptable carrier.

9. A pharmaceutical composition according to claim 8 which further comprises a penicillin or cephalosporin.

10. A compound as claimed in any one of claims 1 to 5 for use in the treatment of bacterial infection.

European Patent Office

# EUROPEAN SEARCH REPORT

0086590

Application number

EP 83 30 0495

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 004 132 (BEECHAM)<br>*Claims*<br>--- | 1,2,6,<br>8 | C 07 D 487/04<br>A 61 K 31/40 //<br>C 07 C 103/48<br>(C 07 D 487/04<br>C 07 D 209/00<br>C 07 D 205/00 )<br>(A 61 K 31/40<br>A 61 K 31/43 )<br>(A 61 K 31/40<br>A 61 K 31/545) |
| A | EP-A-0 028 778 (MERCK)<br>*Claims*<br>----- | 1,2,9 | |
| | | | TECHNICAL FIELDS SEARCHED (Int. Cl. 3) |
| | | | C 07 D 487/00<br>A 61 K 31/00 |

The present search report has been drawn up for all claims

| Place of search | Date of completion of the search | Examiner |
|---|---|---|
| THE HAGUE | 15-04-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document